Europäisches Patentamt

European Patent Office

Office européen des brevets

(11) Publication number: **0 433 311 B1**

(12) **EUROPEAN PATENT SPECIFICATION**

(45) Date of publication of patent specification: **13.04.94**  (51) Int. Cl.5: **G01N 27/10**, G01N 23/08

(21) Application number: **89909630.9**

(22) Date of filing: **30.08.89**

(86) International application number:
**PCT/NO89/00088**

(87) International publication number:
**WO 90/02941 (22.03.90 90/07)**

(54) **PROCESS AND INSTRUMENT FOR A THREE COMPONENT MEASUREMENT.**

(30) Priority: **01.09.88 GB 8820687**

(43) Date of publication of application:
**26.06.91 Bulletin 91/26**

(45) Publication of the grant of the patent:
**13.04.94 Bulletin 94/15**

(84) Designated Contracting States:
**AT BE CH DE FR GB IT LI LU NL SE**

(56) References cited:
**DE-A- 1 798 370       DE-C- 2 533 943
GB-A- 2 088 050       US-A- 3 675 121
US-A- 4 644 263       US-A- 4 713 603**

**J. Phys. E: Sci. Instrum., Vol. 18, 1985 (Great Britain) Eivind Dykesteen et al: "Non-intrusive three-component ratio measurement using an impedance sensor"**

(73) Proprietor: **CHR. MICHELSENS INSTITUTT
Fantoftvn. 38
N-5036 Fantoft(NO)**

(72) Inventor: **HAMMER, Erling
Saudalskleivane 66
N-5088 Mjolkeraen(NO)**
Inventor: **DYKESTEEN, Eivind
Sandslikroken 10
N-5049 Sandsli(NO)**

(74) Representative: **Rostovanyi, Peter et al
AWAPATENT AB
Box 5117
S-200 71 Malmö (SE)**

EP 0 433 311 B1

## Description

This invention relates to a process and an instrument for determining the proportions of the phases of gas, water and oil in a gas/water/oil mixture.

This invention is applicable on different types of three-phase mixtures, but will herein be described mainly with reference to a three-phase mixture containing gas (g), water (w) and oil (o). Accordingly, the equations stated herein are referring to a three-phase mixture containing gas, water and oil and to the three specific phases thereof. However, the equations stated herein are generic to different phases i.e. in general to three phases stated as phase g, w, and o, respectively.

In crude oil production, the fluid at the well head rarely, if ever, involves single phase single component flow. Usually it will contain crude oil, gas (in the free state and/or dissolved in the oil) and possibly water with the proportion of the latter tending to increase as production continues.

Measurements of the rates of hydrocarbon production from the wells in a field are required for reservoir management and production allocation. With such data, depletion of the reservoir can be undertaken with the aim of optimising total production over the field life. In addition the metering of the total production rate of oil and gas from a field is required for fiscal purposes. This demands a much higher degree of accuracy.

At present, crude oil production systems require separation of the gas, water and oil phases before satisfactory metering, for whatever reason, can be achieved.

According to Journal of physics E: Scientific Instruments June 1985 Vol. 18 No. 6 pages 540-544 GB. Evind Dykesteen et al: "Non-intrusive three-component ratio measurement using an impedance sensor", the proportions of the oil/gas/water mixture are determined by capacitance and resistivity measurements (via permittivity).

US-A-3675121 discloses the measurement of proportions of an oil/gas/water mixture by removing the gas and part of the water and then determining the proportion of water and oil by measuring the real part of $E_b$.

GB-A-2088050 discloses analyses of a multicomponent material having at least three components, using gamma radiation having at least two different energies.

Offshore production platforms of today, e.g. those in the North Sea, have a manifold system which allows the flow from a production well to be directed either to the main separators or to a test separator.

Thus at any time, although usually to a set cycle, the flow from a single well can be directed to a test separator where it is separated into gas, water and oil. Meanwhile the production from all other wells is manifolded together and processed in the main separation system. The flowrates of gas, water and oil are measured in the respective single phase lines from the test separator. All three flows are normally measured by using orifice plates, turbine meters or other conventional equipment. A considerable proportion of remaining oil reserves is believed to lie offshore under water depths in excess of 200 metres, in relatively small oil fields, and in hostile environments. As any one of these conditions intensifies, and more particularly when two or more are present together, the cost of conventional offshore recovery systems wherein drilling and production facilities are mounted on surface platforms rises rapidly and soon becomes uneconomic.

For this reason attention has been given to subsea systems where a favoured technique is to drill a number of wells close together and to mount the well head control equipment on the sea bed.

In any design for a new production facility the need for metering of the flows from individual wells, and of the bulk output of the field, must be considered in detail. With the advent of alternative production system concepts such as those outlined above it has become apparent that new methods of flow measurement are desirable to improve both the technical and economic viability of such projects.

One particular problem with multi-phase regimes is the variability of flow conditions in the line. Stratified flow, wavy flow, bubble flow, plug flow, slug flow and annular flow can all occur at various times in horisontal lines. Vertical flow avoids stratification, however.

The density of a three-phase gas/water/oil mixture is given by:

$$\rho_m = \alpha\rho_g + \beta\rho_w + (1-\alpha-\beta)\rho_o \qquad [1]$$

where $\rho_m$ is the density of the three-phase mixture, $\rho_g$ is the gas density, $\rho_w$ is the water density and $\rho_o$ is the oil density. This $\rho_m$ is the density that is measured by a gamma densitometer. If we somehow know the water fraction $\beta$, we can calculate the gas fraction $\alpha$ from Eq. 1 as follows:

2

EP 0 433 311 B1

$$\alpha = \frac{\rho_m - \beta(\rho_w - \rho_o) - \rho_o}{(\rho_g - \rho_o)} \qquad [2]$$

Conversely if the gas fraction is known, Eq. 1 can be used to compute the water fraction $\beta$. From this it is obvious that a gamma densitometer alone cannot be used to measure either the gas or water fraction.

The permittivity of a three phase mixture can be related to the phase volume fractions by the following relationship:

$$\frac{1 - \epsilon_b}{1 - \epsilon_s} \left(\frac{\epsilon_s}{\epsilon_b}\right)^{1/3} = 1 - \alpha \qquad [3]$$

where $\alpha$ is the gas fraction, and $\epsilon_b$ is permittivity of the three-phase mixture. $\epsilon_s$ is the permittivity of a two-phase, i.e. oil/water phase of the three-phase mixture, and is given by

$$\epsilon_s = \frac{\epsilon_o}{(1 - \beta_f)^{3-4.2 \cdot \beta_f^6 + 3 \beta_f^{10}}} \qquad [3a]$$

where $\beta_f$ is the water concentration in the two-phase, i.e. oil/water phase. This formula is a slight modification of Bruggemans formula for two-phase mixtures. The actual water fraction of the three-phase mixture is given by:

$$\beta = (1-\alpha) \beta_f \qquad [3b]$$

According to the present invention there is provided a process for determining the proportions of gas, water and oil in a gas/water/oil mixture. The process of the invention is characterised in, during passing the three-phase mixture in a flow through a spacing between two opposite electrodes (21,22), measuring the permittivity of said flowing mixture by a capacitive non-penetrating first sensor (11) and the density of said flowing mixture by a second sensor (15) including a radioactive element and a gamma radiation densitometer (17), processing electronic signals received from said sensors (11,15) in a computer by incorporating the following equations therein:

$$\frac{1 - \epsilon_b}{1 - \epsilon_s} \left(\frac{\epsilon_s}{\epsilon_b}\right)^{1/3} = 1 - \alpha \qquad [3]$$

where $\alpha$ is the fraction of a first phase, i.e. gas, in the three phase mixture; $\epsilon_b$ is the permittivity of the three-phase mixture, $\epsilon_s$ is the permittivity of a two-phase mixture of a second (water) and a third (oil) phase, i.e. water and a third phase, i.e. oil, and is given by:

$$\epsilon_s = \frac{\epsilon_o}{(1 - \beta_f)^{3-4.2 \cdot \beta_f^6 + 3 \beta_f^{10}}} \qquad [3a]$$

where $\epsilon_o$ is the permittivity of the third phase i.e. oil; and where $\beta_f$ is the concentration of the second phase in said two-phase mixture and the actual fraction of the second phase in the three-phase mixture is given by:

3

$$\beta = (l-\alpha)\,\beta_f \qquad [3b]$$

and

$$\alpha = \frac{\rho_m - \beta(\rho_w - \rho_o) - \rho_o}{(\rho_g - \rho_o)} \qquad [2]$$

where $\rho_g$ is the gas density, $\rho_w$ is the water density, $\rho_o$ is the oil density, and $\rho_m$ is the density that is measured by the gamma-radiation densitometer, and

iteratively determining the proportions of said three phases in said three-phase mixture.

It is thus possible to determine in succession and with a adequate accuracy the three components of the mixture flow at rather short intervalls during the passage of the mixture flow in the spacing between the electrodes.

Further, according to the present invention there is provided an instrument for determining the proportions of gas, water and oil in a gas/water/oil mixture. The instrument is characterised by a non-intrusive measurement instrument comprising

a) a first electrode impedance capacitance sensor, including two opposite electrodes (21,22) located at opposite sides of an intermediate concentrated flow of said three-phase mixture for measuring the permittivity of said three-phase mixture,

b) an impedance sensor head (20) for reducing or eliminating impedance between the electrodes, including an outer screen(26) surrounding an insulation (27) incorporating the two opposite electrodes (21,22) and a guard (25).

c) a density meter (15) including a radioactive element (16) and a gamma radiation densitometer (17) for determining the density of the mixture, and

d) a computer (14) for processing the electronic signals and determining the proportions of said three phases, i.e. gas, water and oil, in said three-phase mixture.

The algorithm in the computer of the instrument of the present invention will be like this:

1. Choose a value for $\beta$.

2. Calculate $\alpha$ using $\beta$ and the measured density of the mixture (Eq. 2).

3. From $\alpha$ and the measured permittivity of the mixture, calculate a new value from $\beta$ using the three-phase permittivity formula (Eq.3).

4. Go to step 2 and repeat the process a predetermined number of times, or use some convergence criteria to stop the iteration. The accuracy of the final result depends on a number of factors. These are briefly discussed below.

(a) The accuracy depends strongly on how accurate the densitiy of the crude oil is known. The density depends on the temperature and pressure, and therefore it will be necessary to compensate for temperature and pressure variations. The density changes with pressure because of the compressibility of the oil, and also because of changing gas/oil-ratio with changing pressure.

(b) The density of the water must also be known with high precision. The water density depends strongly on the salinity, and therefore it has to be measured in each case. For water the variation in density with respect to temperature and pressure probably can be ignored for reasonable temperature and pressure variations.

(c) The density of the gas also has to be known, and also this quantity depends on temperature, pressure and on its composition. At atmospheric pressure the gas density will only be about $1\,kg/m^3$, and can be neglected compared to the densities of oil and water. At substantially higher pressures this can not be neglected and compensation must be made.

(d) The permittivity of the crude oil should be known within 1% in order to avoid significant errors because of this factor.

(e) Finally the accuracy will depend on the accuracy of the densitometer and the capacitance measurement.

Situating the sensor inline in the production system means that very little pressure drop can be tolerated across the sensor. Furthermore in a riser, erosion can be very high and therefore the sensor is non-intrusive.

Taking these factors into account the electrodes of the sensor head are in the shape of arcuate electrode plates incorporated in a tube forming sensor head each of said electrodes facing the mixture flow in said sensor head. In the following the sensor is denoted as a surface plate sensor.

The sensor head is ring shaped or sleeve shaped and incorporates a pair of radially innermost electrodes and a radially outermost screen and an intermediate volume containing electric insulating material. The screen is at earth potential. The arcuate electrode plates may be spaced from the mixture flow by a layer of electrically insulated material.

In the surface plate electrode sensor, the electrical field penetrates through the whole measuring section, making the sensor sensitive to the flow in the centre of the pipe as well as along the pipe wall. Careful choice of the electrode opening angle ensures that every part of the measuring volume will have the same effect on total sensor impedance.

It can be shown that with respect to homogeneity of the electric field, the optimum electrode opening angle is between 60° and 90°. The sensitivity of a surface plate sensor with such an opening angle will also be good.

In accordance with a preferred embodiment of the invention the electrodes, located circumferencially along the inner surface of the tube forming sensor head, are mutually spaced by an opening angle of between approximately 60 to 90°.

The sensor head also reduces or eliminates impedance between the electrodes. One way of achieving this is by providing the sensor head in each spacing between the electrodes with a strip shaped guard with an extension of some few arcuate degrees, and by connecting said guard strips at the potential of said first electrode.

It is preferred that said guard strips are incorporated in a ring shaped guard surrounding the edges of one of said two electrodes.

Suitable electrically insulating material is a ceramic or a polyurethane or other material having good resistance to erosion and low thermal expansion.

The electrodes are made of an electrically conducting material including stainless steel, conducting ceramic and other suitable material.

The present invention, i.e. the process as well as the instrument of the invention, has the following advantages:

- non intrusive -
- measure complete flow through pipe, no sampling -
- real time -
- no moving parts -
- reliable with low maintenance.

The invention is illustrated by, but not limited with reference to Figures 1 to 4 of the accompanying drawings wherein:

Figure 1 is a schematic representation of a system incorporating a capacitance sensor and a density sensor, i.e. a gamma-densitometer to calculate the flow composition.

Figure 2 is a capacitance measurement transmitter illustrated in Figure 1.

Figure 3 is a section through a sensor.

Figure 4 is a plane view of one of the electrodes and its associated guard.

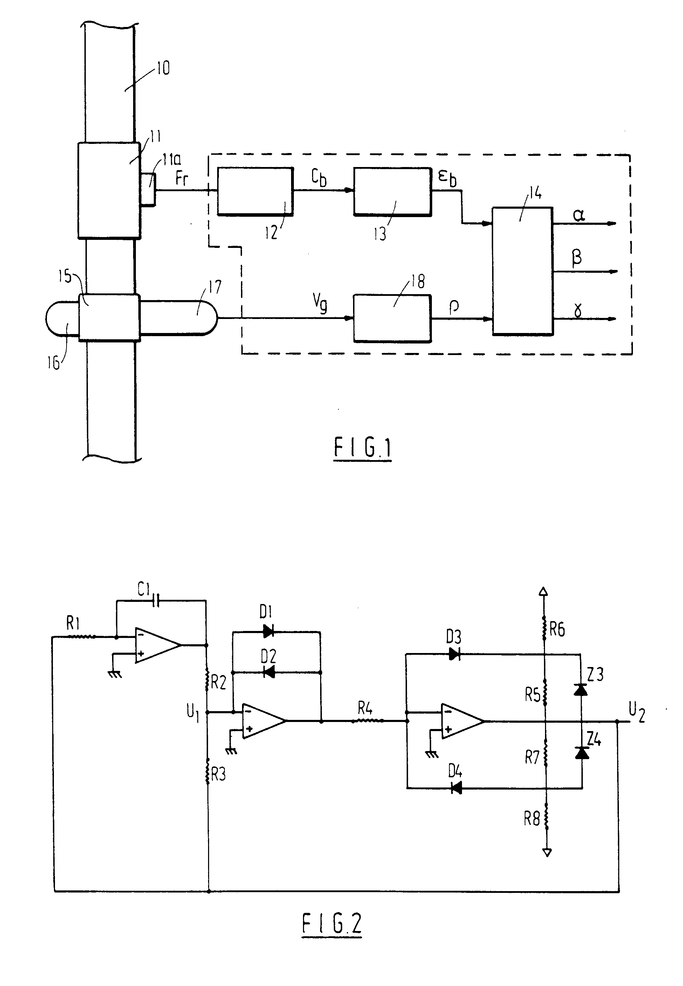

With reference to Figure 1, a three-phase mixture of gas, water and oil flows through a line 10 incorporating a capacitance sensor 11, i.e. a first sensor, which measures the permittivity of the mixture. A transmitter 11a feeds signals from the first sensor 11 to a first data processing unit 12 and further to a second data processing unit 13 incorporated in a computer 14. Said units can incorporate a display of the relative proportions of the three-phase mixture.

A second sensor 15, i.e. a gamma densitometer, is incorporated in the flowline 10 in the vicinity of the first sensor 11. The gamma densitometer 15 include a gamma meter radioactive source 16. Herein a ceasium isotope is selected, but other isotopes such as americium isotope could also be used. Further, the gamma densitometer 15 include a gamma meter detector 17. The gamma meter radioactive source 16 and the gamma meter detector 17 are located at opposite sides of the flowline 10 clamped to a pipe forming sensor head of said second sensor 15. The output of the gamma densitometer, in combination with the output of the first sensor 11, is used to calculate the fractions of the mixture and an accurate result may be obtained rather easily.

The output frequency $F_r$ from the capacitance measurements electronics is converted from a first data processing unit 12 into a digital value $C_b$ which represents the sensor capacitance. The relationship between $C_b$ and $F_r$ is established through calibration of the measurement electronics, and the calibration curve is stored in the computer memory. The computer also stores a calibration curve for the capacitance sensor 11. This is herein used to convert the measured capacitance $C_b$ in a second data processing unit 13 into a value for the permittivity of the mixture, $\epsilon_b$.

The detector unit 17 of the second sensor 15 gives out a signal $V_g$, which is proportional to the density of the mixture flowing inside the pipe. A data processing unit 18 of said computer is converting the signal $V_g$ to the digital value which is the measured density ($\rho_m$) of the flowing mixture. This value from unit 18 and the value $\epsilon_b$ from unit 13 is further processed in a common unit 14 of the computer. The unit 14 contains a mathematical model relating measured permittivity and measured density of the flowing mixture, to the proportions of gas ($\alpha$), water ($\beta$) and oil ($\gamma$).

The capacitance transmitter 11a is illustrated in further details in Figure 2. The sensor capacitance $C_1$ provides a triangular wave $v_1$ which is generated by integration of a square wave $v_2$. In turn this triangular wave $v_1$ is used as the input to a square wave generator. The illustrated closed loop constitutes a sesonance system where the resonance frequency f is given by equation 5:

$$f = k \frac{1}{C_1}$$

wherein k is a constant determined by $R_1$ and zener diodes $Z_3$ and $Z_4$ and $C_1$ is the sensor capacitance.

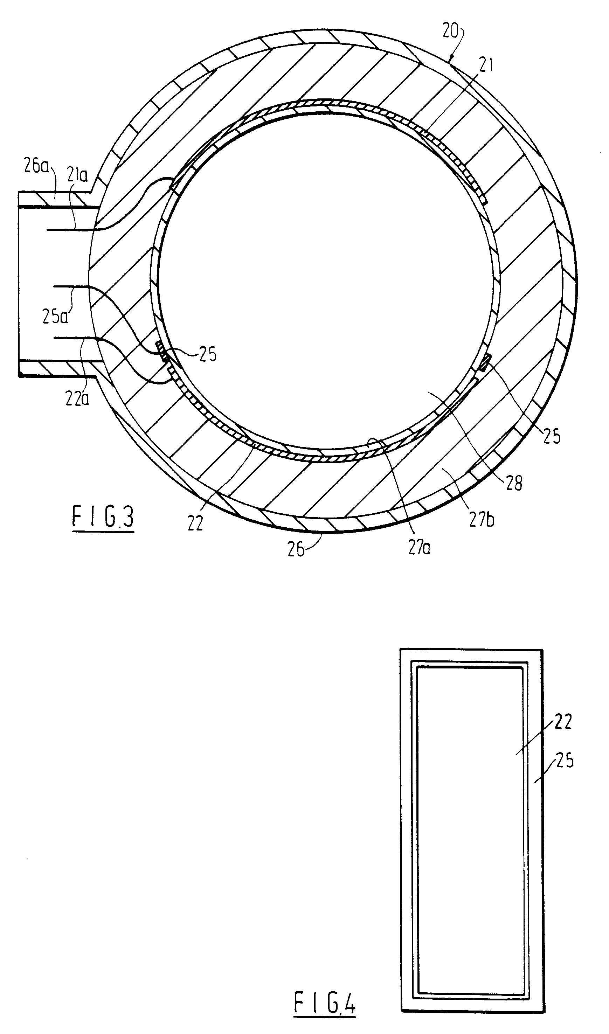

With reference to Figure 3 there is illustrated a section of a sensor head 20 of the first sensor 11. This sensor head 20, being in the shape of a tube forming sensor head, comprises two electrodes 21 and 22, being in the shape of arcuate electrode plates, connected to terminals 21a and 22a and a guard 25 connected to a terminal 25a. An outer screen 26 made of steel is connected to earth and provides a housing for the sensor head and surrounds an insulation 27 wherein there is incorporated the electrodes 21,22 and the guard 25. The insulation 27 is leaving a central cavity 28 for fluid flow.

More detailed the electrodes 21,22 and the guard 25 are insulated from the fluid flow by means of a radially innermost insulation layer 27a made of a first ceramic material, such as sintered alumina (aluminium oxyd). In the spacing between said radially innermost insulation layer 27a and the radially outermost screen 26 there is a main insulation layer 27b made of a second ceramic material, such as "Ceramite"® delivered from Elkem Materials A/S, Norway.

In practice the insulation layer 27b, the electrodes 21,22 and the guard 25 is being enveloped between the inner insulation layer 27a and the outer screen 26. As illustrated in Figure 3 connections 21a,22a,25a are passing from its associated electrode 21,22 and guard 25 respectively via the insulation layer 27b to a connecting box 26a at one side portion of the screen 26. The guard 25 is at the potential of a first one 21 of the two electrodes 21,22.

In Figure 4 is illustrated the electrode 22 and the guard 25 in a plane folder position. The electrode 22 (as well as the electrode 21) is of rectangular shape. It is evident from Figure 4 that the guard 25 is rectangularly ring shaped and spaced from the electrode 22 by a minor gap of say 5 mm. The electrodes 21,22 and the guard 25 are in a preferred embodiment, during production thereof, applied in a rather thin walled layer directly at the radially outer surface of the radially innermost insulation layer 27a and is hence covered by the radially outermost insulation layer 27b.

## Claims

**1.** Process for determining the proportions of the phases of gas, water and oil in a gas/water/oil mixture, **characterised in**

during passing the three-phase mixture in a flow through a spacing between two opposite electrodes (21,22), measuring the permittivity ($\epsilon_b$) of said flowing mixture by a capacitive non-penetrating first sensor (11), and the density ($\rho_m$) of said flowing mixture by a second sensor (15) including a radioactive element (16) and a gamma radiation densitometer (17),

processing electronic signals received from said sensors (11,15) in a computer (14) by incorporating the following equations therein:

$$\frac{1 - \epsilon_b}{1 - \epsilon_s} \left( \frac{\epsilon_s}{\epsilon_b} \right)^{1/3} = 1 - \alpha \qquad [3]$$

where $\alpha$ is the fraction of a first phase, i.e. gas, in the three phase mixture; $\epsilon_b$ is the permittivity of the

three-phase mixture, $\epsilon_s$ is the permittivity of a two-phase mixture of a second phase, i.e. water, and a third phase, i.e. oil and is given by:

$$\epsilon_s = \frac{\epsilon_o}{(1 - \beta_f)^{3-4.2 \cdot \beta_f^6 + 3\beta_f^{10}}} \qquad [3a]$$

where $\epsilon_o$ is the permittivity of the third phase i.e. oil; and
where $\beta_f$ is the concentration of the second phase in said two-phase mixture and the actual fraction of the second phase in the three-phase mixture is given by:

$$\beta = (l-\alpha)\,\beta_f \qquad [3b];$$

and

$$\alpha = \frac{\rho_m - \beta(\rho_w - \rho_o) - \rho_o}{(\rho_g - \rho_o)} \qquad [2]$$

where $\rho_g$ is the gas density, $\rho_w$ is the water density, $\rho_o$ is the oil density, and $\rho_m$ is the density that is measured by the gamma-radiation densitometer (17), and
   iteratively determining the proportions of said three phases in said three-phase mixture.

2. Instrument for determining the proportions of phases gas, water and oil in a gas/water/oil mixture, comprising
   a non-intrusive measurement instrument which comprises
   a) a first electrode impedance capacitance sensor, including two opposite (21,22) electrodes located at opposite sides of an intermediate concentrated flow of said three-phase mixture, and characterised by further comprising:
   b) an impedance sensor head (20) for reducing or eliminating impedance between the electrodes, including an outer screen (26) surrounding an insulation (27) incorporating the two opposite electrodes (21,22) and a guard (25).
   c) a density meter (15) including a radioactive element (16) and a gamma radiation densitometer (17) for determining the density of the mixture, and
   d) a computer (14) for processing the electronic signals and determining the proportions of said three phases, i.e. gas, water and oil, in said three-phase mixture
   and wherein the first electrode impedance capacitance Sensor is for measuring the permittivity ($\epsilon_b$) of said three-phase mixture

3. Instrument according to claim 2, **characterised in**
   that the electrodes (21,22) of the sensor head (20) are in the shape of arcuate electrode plates incorporated in a tube forming sensor head (20), each of said electrodes (21,22) facing the mixture flow in said sensor head (20).

4. Instrument according to one of claims 2 or 3, **characterised in**
   that the sensor head (20) is ring shaped or sleeve shaped and incorporates a pair of radially innermost electrodes (21,22) and a radially outermost screen (26) and an intermediate volume containing electrically insulating material (27).

5. Instrument according to one of claims 3 to 4, **characterised in**
   that the screen (26) is at earth potential.

6. Instrument according to one of claims 3 to 5, **characterised in**
   that the arcuate electrode plates (21,22) are spaced from the mixture flow by a layer (27a) of

7

electrically insulated material.

7. Instrument according to one of claims 3 to 6, **characterised in**
   that the electrodes (21,22) located circumferencially along the inner surface of the tube forming sensor head (20), are mutually spaced by an opening angle between approximately 60 to 90°.

8. Instrument according to claims 5 and 7, **characterised in**
   that the sensor head (20) in each spacing between the electrodes (21,22) is provided with a strip shaped guard (25) with an extension of some few arcuate degrees, and
   that said guard strips (25) are at the potential of a first one (21) of the electrodes (21,22).

9. Instrument according to claim 8, **characterised in**
   that said guard strips are incorporated in a ring shaped guard (25) surrounding the edges of one (22) of said two electrodes (21,22).

10. Instrument according to one of claim 4 and 6, **characterised in**
    that the electrically insulating material (27) is ceramic.

11. Instrument according to one of claims 2 to 10, **characterised in**
    that the electrodes (21,22) are made of an electrically conducting ceramic.

## Patentansprüche

1. Verfahren zur Feststellung der Proportionen der Phasen von Gas, Wasser und Öl in einem Gas/Wasser/Öl-Gemisch, **gekennzeichnet** durch die folgenden Schritte:
   Messen, während das Dreiphasengemisch in einem Strom durch einen Zwischenraum zwischen zwei entgegengesetzten Elektroden (21,22) geleitet wird, der Permittivität ($\epsilon_b$) des strömenden Gemisches mit einem kapazitiven nichtdurchdringenden ersten Messfühler (11) und der Dichte ($\rho_m$) des strömenden Gemisches mit einem zweiten Messfühler (15), der ein radioaktives Element (16) und ein Gammastrahlungsdensitometer (17) aufweist;
   Bearbeiten der von den genannten Messfühlern (11,15) empfangenen elektronischen Signale in einem Computer (14) durch Einlagerung der folgenden Gleichungen:

$$\frac{1 - \epsilon_b}{1 - \epsilon_s} \left( \frac{\epsilon_s}{\epsilon_b} \right)^{1/3} = 1 - \alpha \qquad [3]$$

worin $\alpha$ die Fraktion einer ersten Phase, d.h. Gas, des Dreiphasengemisches ist; $\epsilon_b$ die Permittivität des Dreiphasengemisches, $\epsilon_s$ die Permittivität eines Zweiphasengemisches einer zweiten Phase, d.h. Wasser, und einer dritten Phase, d.h. Öl, ist und durch folgende Formel gegeben ist:

$$\epsilon_s = \frac{\epsilon_o}{(1 - \beta_f)^{3-4.2} \cdot \beta_f^{6} + 3 \, \beta_f^{10}} \qquad [3a]$$

worin $\epsilon_o$ die Permittivität der dritten Phase, d.h. Öl, ist; und worin $\beta_f$ die Konzentration der zweiten Phase des genannten Zweiphasengemisches ist und die tatsächliche Fraktion der zweiten Phase des Dreiphasengemisches durch folgende Formel gegeben ist:

$$\beta = (1-\alpha) \, \beta_f \qquad [3b]$$

und

$$\alpha = \frac{\rho_m - \beta(\rho_w - \rho_o) - \rho_o}{(\rho_g - \rho_o)} \qquad [2]$$

worin $\rho_g$ die Gasdichte, $\rho_w$ die Wasserdichte, $\rho_o$ die Öldichte, und $\rho_m$ die mit dem Gammastrahlungs-densitometer (17) gemessene Dichte ist, und

iteratives Feststellen der Proportionen der genannten drei Phasen im Dreiphasengemisch.

2. Instrument zur Feststellung der Proportionen der Gas-, Wasser- und Ölphasen eines Gas/Wasser/Öl-Gemisches, umfassend

ein nichtintrusives Messinstrument, umfassend

a) einen ersten Elektroden-Impedanz/Kapazitätsmessfühler mit zwei entgegengesetzten (21,22) Elektroden, die auf entgegengesetzten Seiten eines dazwischen sich bewegenden, konzentrierten Stroms des Dreiphasengemisches angeordnet sind, und dadurch **gekennzeichnet**, dass es ferner folgende Mittel umfasst:

b) einen Impedanzmessfühlerkopf (20) zum Reduzieren oder Beseitigen von Impedanz zwischen den Elektroden, mit einer Aussenabschirmung (26), die eine Isolierung (27) mit den beiden entgegengesetzten Elektroden (21, 22) und einem Wächter (25) umschliesst,

c) einen Dichtemesser (15) mit einem radioaktiven Element (16) und einem Gammastrahlungsdensitometer (17) zur Feststellung der Dichte des Gemisches, und

d) einen Computer (14) zur Bearbeitung der elektronischen Signale und Feststellung der Proportionen der genannten drei Phasen, d.h. Gas, Wasser und Öl, des Dreiphasengemisches,

und wobei der erste Elektroden-Impedanz/Kapazitätsmessfühler zum Messen der Permittivität ($\epsilon_b$) des Dreiphasengemisches vorgesehen ist.

3. Instrument nach Anspruch 2, dadurch **gekennzeichnet**,

dass die Elektroden (21,22) des Messfühlerkopfes (20) die Form von gebogenen Elektrodenplatten haben, die in einem rohrbildenden Messfühlerkopf (20) enthalten sind, wobei jede Elektrode (21,22) dem Gemischstrom im Messfühlerkopf (20) zugewandt ist.

4. Instrument nach einem der Ansprüche 2 und 3, dadurch **gekennzeichnet**,

dass der Messfühlerkopf (20) ringförmig oder hülsenförmig ist und ein radial innerstes Elektrodenpaar (21,22) und eine radial äusserste Abschirmung (26) sowie ein Zwischenvolumen einschliesst, das ein elektrisch isolierendes Material (27) enthält.

5. Instrument nach einem der Ansprüche 3-4, dadurch **gekennzeichnet**,

dass die Abschirmung (26) Erdpotential hat.

6. Instrument nach einem der Ansprüche 3-5, dadurch **gekennzeichnet**,

dass sich die bogenförmigen Elektrodenplatten (21,22) durch eine Schicht (27a) elektrisch isolierenden Materials im Abstand von dem Gemischstrom befinden.

7. Instrument nach einem der Ansprüche 3-6, dadurch **gekennzeichnet**,

dass die Elektroden (21,22), die in Umfangsrichtung längs der Innenfläche des rohrbildenden Messfühlerkopfes (20) liegen, mit Zwischenraum (Öffnungswinkel von etwa 60 bis 90°) angeordnet sind.

8. Instrument nach den Ansprüchen 5 und 7, dadurch **gekennzeichnet**,

dass der Messfühlerkopf (20) in jedem Zwischenraum zwischen den Elektroden (21,22) einen streifenförmigen Wächter (25) mit einer Ausdehnung von einigen bogenförmigen Graden aufweist, und

dass die Wächterstreifen (25) das Potential der ersten (21) der Elektroden (21,22) hat.

9. Instrument nach Anspruch 8, dadurch **gekennzeichnet**,

dass die Wächterstreifen Teile eines ringförmigen Wächters (25) sind, der die Kanten einer (22) der beiden Elektroden (21,22) umschliesst.

**10.** Instrument nach einem der Ansprüche 4 und 6, dadurch **gekennzeichnet**,
dass das elektrisch isolierende Material (27) keramisch ist.

**11.** Instrument nach einem der Ansprüche 2-10, dadurch **gekennzeichnet**,
dass die Elektroden (21,22) aus einem stromleitenden keramischen Werkstoff hergestellt sind.

## Revendications

**1.** Procédé pour déterminer les proportions des phases de gaz, d'eau et d'huile d'un mélange à trois phases, **caractérisé** en ce que

l'on mesure, pendant l'écoulement du mélange à trois phases à travers un espace entre deux électrodes opposées (21, 22), la permittivité ($\epsilon_b$) du mélange coulant, au moyen d'un premier capteur capacitif non pénétrant (11), ainsi que la densité ($\rho_m$) du mélange coulant au moyen d'un second capteur (15) comprenant un élément radioactif (16) et un densiomètre de rayonnement gamma (17),

l'on traite des signaux électroniques reçus desdits capteurs (11, 15) dans un ordinateur (14) en y incorporant les équations suivantes:

$$\frac{1 - \epsilon_b}{1 - \epsilon_s} \left( \frac{\epsilon_s}{\epsilon_b} \right)^{1/3} = 1 - \alpha \qquad [3]$$

dans laquelle $\alpha$ représente la fraction d'une première phase, c'est-à-dire le gaz, du mélange à trois phases, $\epsilon_b$ représente la permittivité du mélange à trois phases, et $\epsilon_s$ représente la permittivité d'un mélange à deux phases comprenant une deuxième phase, c'est-à-dire l'eau, et une troisième phase, c'est-à-dire l'huile, et est donné par

$$\epsilon_s = \frac{\epsilon_o}{(1 - \beta_f)^{3-4.2 \cdot \beta_f^6 + 3\beta_f^{10}}} \qquad [3a]$$

dans laquelle $\epsilon_o$ représente la permittivité de la troisième phase, c'est-à-dire l'huile, et $\beta_f$ représente la concentration de la deuxième phase dudit mélange à deux phases, et la fraction réelle de la deuxième phase du mélange à trois phases est donnée par

$$\beta = (1-\alpha) \beta_f \qquad [3b]$$

et

$$\alpha = \frac{\rho_m - \beta(\rho_w - \rho_o) - \rho_o}{(\rho_g - \rho_o)} \qquad [2]$$

dans laquelle $\rho_g$ représente la densité du gaz, $\rho_w$ représente la densité de l'eau, $\rho_o$ représente la densité de l'huile, et $\rho_m$ représente la densité mesurée au moyen du densiomètre de rayonnement gamma (17), et

l'on détermine de façon itérative les proportions desdites trois phases du mélange à trois phases.

**2.** Instrument pour déterminer les proportions des phases de gaz, d'eau et d'huile d'un mélange à trois phases, comprenant un instrument de mesure non pénétrant qui comporte

a) un premier capteur d'impédance/capacitance d'électrode comprenant deux électrodes opposées (21, 22) se trouvant des côtés opposés d'un écoulement concentré intermédiaire dudit mélange à trois phases, **caractérisé** en ce qu'il comporte en outre:

b) une tête de capteur d'impédance (20) destinée à réduire ou à supprimer l'impédance entre les électrodes et comportant un écran extérieur (26) qui entoure un isolateur (27) où se trouvent incorporées les deux électrodes opposées (21, 22) et un dispositif protecteur (25),

c) un densiomètre (15) comportant un élément radioactif (16) et un densiomètre de rayonnement gamma (17) destiné à déterminer la densité du mélange, et

d) un ordinateur (14) destiné à traiter les signaux électroniques et à déterminer les proportions desdites trois phases, c'est-à-dire de gaz, d'eau et d'huile, du mélange à trois phases,

le premier capteur d'impédance/capacitance d'électrode étant prévu pour mesurer la permittivité ($\epsilon_b$) du mélange à trois phases.

3. Instrument selon la revendication 2, **caractérisé** en ce que

les électrodes (21, 22) de la tête de capteur (20) sont des plaques d'électrode en forme d'arc se trouvant incorporées dans la tête de capteur tubulaire (20), chacune des électrodes (21, 22) faisant face à l'écoulement du mélange dans ladite tête de capteur (20).

4. Instrument selon la revendication 2 ou 3, **caractérisé** en ce que

la tête de capteur (20) est annulaire ou sous forme de manchon et comporte une paire d'électrodes radialement les plus internes (21, 22), un écran radialement le plus externe (26) et un espace intermédiaire contenant du matériau électriquement isolant (27).

5. Instrument selon la revendication 3 ou 4, **caractérisé** en ce que

l'écran (26) est au potentiel terrestre.

6. Instrument selon l'une des revendications 3 à 5, **caractérisé** en ce que

les plaques d'électrode en forme d'arc (21, 22) sont écartées de l'écoulement du mélange au moyen d'une couche (27a) de matériau électriquement isolant.

7. Instrument selon l'une des revendications 3 à 6, **caractérisé** en ce que

les électrodes (21, 22), prévues le long de la circonférence de la face interne de la tête de capteur tubulaire (20), sont espacées l'une de l'autre d'un angle d'ouverture d'environ 60-90°.

8. Instrument selon les revendications 5 et 7, **caractérisé** en ce que

la tête de capteur (20), prévue dans chaque espace entre les électrodes (21, 22), est munie d'un dispositif protecteur sous forme de bande (25) ayant une étendue de quelques degrés arqués, et

les bandes protectrices (25) sont au potentiel d'une première (21) des électrodes (21, 22).

9. Instrument selon la revendication 8, **caractérisé** en ce que

les bandes protectrices se trouvent incorporées dans un dispositif protecteur annulaire (25) entourant les bords d'une (22) des deux électrodes (21, 22).

10. Instrument selon la revendication 4 ou 6, **caractérisé** en ce que

le matériau électriquement isolant (27) est la céramique.

11. Instrument selon l'une des revendications 2 à 10, **caractérisé** en ce que

les électrodes (21, 22) sont réalisées en céramique conductrice.

$\underline{F I G.1}$

$\underline{F I G.2}$

EP 0 433 311 B1

FIG.3

FIG.4

13